# EUROPEAN PATENT APPLICATION

(11) **EP 1 738 701 A1**
(43) Date of publication of application: **03.01.2007**
(21) Application number: 06013245.3
(22) Date of filing: 27.06.2006
(51) Int. Cl.: A61B 17/34

(54) **Beveled access apparatus with locking ribs elements**

(30) Priority: 30.06.2005 US 170824
(71) Applicant: Tyco Healthcare Group LP, Norwalk, Connecticut 06856 (US)
(72) Inventor: Smith, Robert C., Cheshire, CT 06410 (US); Jensen, David, Westbrook, CT 06498 (US)
(74) Representative: HOFFMANN EITLE

(57) **Abstract**

A surgical access apparatus includes an access member (100) dimensioned for positioning within body tissue. The access member includes an outer wall (112) defining a longitudinal opening (114) along a longitudinal axis (j) of the access member. The outer wall includes at least one locking element (116) projecting radially outwardly from the outer wall. The at least one locking element defines first (118), second (120) and third (122) surfaces. The first surface is a leading surface and is arranged at a leading angle relative to the longitudinal axis to permit insertion of the access member in the body tissue. The second surface extends from the first surface. The third surface extends from the second surface and is a trailing surface arranged at a trailing angle relative to the longitudinal axis to resist removal of the access member from the body tissue. Preferably, the outer wall of the access member is transparent. The outer wall may include a plurality of locking elements. Adjacent locking elements may be longitudinally spaced relative to the longitudinal axis.

## Description

### TECHNICAL FIELD

The present disclosure generally relates to surgical instruments for performing laparoscopic and endoscopic surgical procedures, and, more particularly, relates to a transparent access apparatus incorporating a retention mechanism for securing the apparatus within an incision in a patient's body.

### BACKGROUND

In laparoscopic and endoscopic surgical procedures, a small incision or puncture is made in the patient's body to provide access for a tube or a cannula device which is inserted into the patient's body to permit viewing of the surgical site, and/or, the insertion of instruments used in performing the surgical procedure. Typically, a trocar assembly, having an obturator with a sharpened tip and a cannula, is used. The obturator is assembled with the cannula and the obturator is used to penetrate the body wall and create the path to the surgical site. When the obturator is removed, the cannula remains in place to maintain access to the surgical site. Several incisions may be made to provide numerous access ports to the surgical objective. Once the cannulas are in place, various surgical instruments such as scissors, dissectors, retractors or the like, may be inserted by a surgeon to perform the surgery. Typically, an endoscope is used to view the area directly, and may incorporate a miniature camera to display the surgical site on a video monitor in the operating room.

In order to maintain the cannula within the incision, it has been known to provide various mechanisms such as external sleeves, expandable members, etc, which engage the tissue surrounding the incision to prevent undesired removal of the cannula. Howevex, such known mechanisms are generally complex in nature. Moreover, these mechanisms oflen are potentially invasive to the surrounding tissue thereby increasing the likelihood of undesired tissue tear which consequently increases patient trauma and recovery time.

### SUMMARY

Accordingly, a surgical access apparatus of the present disclosure includes an access member dimensioned for positioning within body tissue. The access member includes an outer wall defining a longitudinal opening along a longitudinal axis of the access member. The outer wall includes at least one locking element projecting radially outwardly from the outer wall. The at least one locking element defines first, second and third surfaces. The first surface is a leading surface and is arranged at a leading angle relative to the longitudinal axis to permit insertion of the access member in the body tissue. The second surface extends from the first surface. The third surface extends from the second surface and is a trailing surface arranged at a trailing angle relative to the longitudinal axis to resist removal of the access member from the body tissue. Preferably, the outer wall of the access member is transparent.

In one preferred embodiment, the outer wall includes a plurality of locking elements. Adjacent locking elements may be longitudinally spaced relative to the longitudinal axis. The leading angle of the first surface may range from about 5° to about 30° relative to the longitudinal axis, more preferably, about 14° to about 16° relative to the longitudinal axis. The trailing angle is substantially transverse to the longitudinal axis.

The access member may include a beveled leading end. The beveled leading end may include first and second intersecting surfaces. The first and second intersecting surfaces preferably intersect along a median bisecting plane of the access member. Alternatively, the beveled leading end may have an arcuate characteristic,

In another embodiment, a surgical access instrument includes an access member dimensioned for positioning within body tissue and defining a longitudinal axis. The access member has leading and trailing ends. The leading end includes first and second intersecting surfaces arranged with respect to the longitudinal axis at first and second oblique angles. The first and second intersecting surfaces of the leading end intersect along a median bisecting plane of the access member. The first surface of the leading end defines an angle ranging from about 35° to about 75°, preferably, about 55° relative to the longitudinal axis, and the second surface of the leading end defines an angle ranging from about 25° to about 65°, preferably about 45° relative to the longitudinal axis.

The access member includes an outer wall defining a longitudinal opening extending along the longitudinal axis. The outer wall includes at least one locking element projecting radially outwardly from the outer wall. The at least one locking element defines first, second and third surfaces. The first surface is a leading surface and is arranged at a leading angle relative to the longitudinal axis to permit insertion of the access member in the body tissue. The second surface extends from the first surface. The third surface extends from the second surface and is a trailing surface arranged at a trailing angle relative to the longitudinal axis to resist removal of the access member from the body tissue. The outer wall may include a plurality of locking projections. Preferably, adjacent locking projections are longitudinally spaced relative to the longitudinal axis.

### BRIEF DESCRIPTION OF THE DRAWINGS:

Preferred embodiments of the present disclosure will be better appreciated by reference to the drawings wherein:
Figure 1 is a side view of a trocar assembly incorporating a cannula in accordance with the principles of the present disclosure;
Figure 2 is a side plan view of the cannula in accordance with the embodiment of Figure 1;
Figure 3 is a side cross-sectional view of the cannula in accordance with the embodiment of Figures 1-2;
Figure 4 is an enlarged partial plan view illustrating the locking ribs of the cannula in accordance with the embodiment of Figures 1-3;
Figure 5 is a side cross-sectional view of the leading end of the cannula in accordance with the embodiment of Figures 1-4; and
Figure 6 is a side cross-sectional view similar to Figure 5 illustrating leading end of a cannula in accordance with another embodiment of the present disclosure.

### DETAILED DESCRIPTION OF THE PREFERRED EMBODIMENTS:

The present disclosure is particularly suited for use with surgical access devices including cannulas, catheters, endoscopic tubes, sheaths or the like. Such access devices are typically utilized in conjunction with a surgical procedure for introducing/withdrawing fluids or to permit insertion of instrumentation required to satisfactorily perform the surgical procedure. The following description of the present invention will be focused on its use with a surgical trocar or cannula assembly; however, it is appreciated that the present invention has application in any of the surgical access devices of the type listed hereinabove. In addition, the present disclosure may also be adapted to provide access for a physician's arm or hand during a hand-assisted laparoscopic procedure. In this application, the hand access device is introduced within the body to provide access to underlying tissue in, e.g., the abdominal cavity. Thereafter, the physician's arm and/or hand are introduced through the access device to assist in performing the desired procedure.

In the following description, as is traditional, the term "proximal" refers to the portion of the instrument closest to the operator while the term "distal" refers to the portion of the instrument remote from the operator.

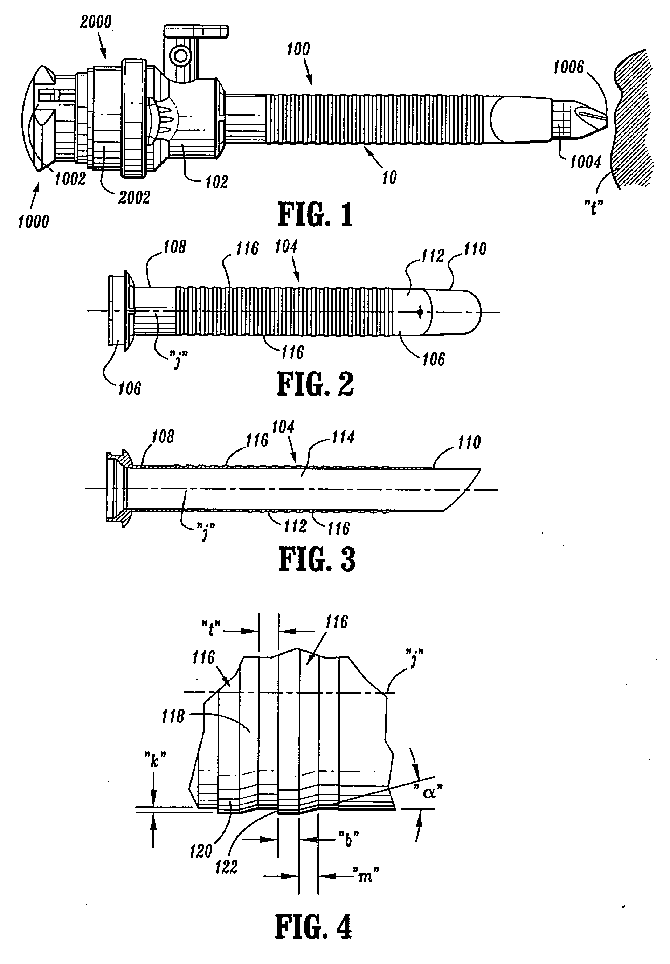

Referring now to the drawings, in which like reference nmnerals identify identical or substantially similar parts throughout the several views, Figure 1 illustrates a surgical trocar assembly 10 which incorporates the principles of the present disclosure. One suitable trocar assembly is disclosed in commonly assigned U.S. Pat. No. 4,601,710 to Moll, the contents of which are incorporated herein by reference. Generally, trocar assembly 10 includes cannula 100 and obturator 1000 which is positionable within the cannula 100. Obturator 1000 includes an obturator housing 1002 and an obturator portion 1004 extending from the obturator housing 1002. Obturator portion 1004 has pointed or penetrating obturator tip 1006 for penetrating tissue. Obturator tip 1006 may be pyramidal in configuration defining sharpened edges along the lines of intersection of the pyramidal blade. Alternatively, obturator tip 1006 may incorporate a flat or planar blade. It is further envisioned that obturator tip 1006 may be blunt, conical, or have another shape. Obturator 1000 is utilized to penetrate the abdominal wall to introduce cannula 100 through the abdominal wall, and then subsequently is removed from the cannula 100 to permit introduction of the surgical instrumentation utilized to perform the procedure through the passageway.

Referring now to Figures 2-3, in conjunction with Figure 1, cannula 100 includes cannula housing 102 and cannula sleeve 104 connected to the housing 102 and extending distally therefrom. Cannula sleeve 104 may be connected to cannula housing 102 through any conventional means including cements, adhesives, bayonet coupling, tongue and groove arrangements, etc. In a preferred embodiment, cannula sleeve incorporates flange 106 which is mounted to corresponding structure of cannula housing 102 by any of the aforementioned conventional means.

Cannula sleeve 104 defines a longitudinal axis "j" extending along the length of the cannula sleeve 104. Cannula sleeve 104 has proximal or trailing end portion 108 and distal or leading end portion 110. Cannula sleeve 104 includes outer wall 112 which defines internal longitudinal passage 114. Longitudinal passage 114 is dimensioned to permit passage of surgical instrumentation. Cannula sleeve 104 may be formed of stainless steel or other rigid materials such as a polymeric material or the like and is desirably a medical grade material. Cannula sleeve 104 may be transparent, translucent or opaque, or may have different portions of different opacity or translucence. In a preferred embodiment, cannula sleeve 104 is transparent to permit viewing through outer wall 112 and into longitudinal passage 114, The diameter of cannula sleeve 104 may vary, but, typically ranges from about 4.5 to about 15 mm.

As best depicted in Figures 2-4, outer wall 112 of cannula sleeve 104 includes a plurality of spaced locking elements or projections 116 extending radially outwardly from the outer wall 112. Locking elements 116 are integrally formed with outer wall 112, and, preferably, monolithically formed during manufacture, e.g., an injection molding process, to define an annular or ring like shaped elements as shown. Locking elements 116 preferably extend about the entire circumference of outer wall 112. It is appreciated, however, that locking elements 116 may extend about only a peripheral portion of outer wall 112, Locking elements 116 are preferably spaced along the longitudinal axis "j" of cannula sleeve 104 with adjacent elements 116 being spaced by a distance "t".

Each locking element 116 defines three contiguous surfaces, namely, first or leading surface 118, second surface 120 extending from the first surface 118 and third or trailing surface 122 which extends from the second surface 120. First surface 118 is arranged at an angle "α" relative to longitudinal axis "j". Angle "α'' is preferably sufficiently oblique to permit and/or facilitate passage of cannula sleeve 104 through tissue. In a preferred embodiment, angle "α" extends at an angle ranging from about 5° to about 30° relative to longitudinal axis "j" and, more preferably, about 14° to about 16°. First surface 118 may define a longitudinal length "m" which ranges from about .025 inches to about .075 inches and, preferably, about .050 inches. Second surface 120 is preferably substantially parallel to the longitudinal axis "j" of cannula sleeve 104. However, it is envisioned that second surface 120 may be slightly oblique with respect to the longitudinal axis "j". Second surface 120 defines a longitudinal length "b" varying from about .025 inches to about .075 inches and, more preferably, about .050 inches.

Third surface 122 is also arranged at an angle relative to longitudinal axis "j". Preferably, third surface 122 is transverse or arranged at about a 90° angle relative to the longitudinal axis "j". It is envisioned that third surface 122 also may be obliquely arranged relative to the axis "j". Third surface 122 is particularly configured to resist the tendency of cannula sleeve 104 to move in a withdrawal or proximal direction thus facilitating retention of cannula sleeve 104 within the tissue. Moreover, third surface 122 is configured to engage the tissue in a manner to prevent "retropulsion" of sleeve 104 relative to tissue. Third surface 122 defines a height "k" ranging from about .01 inches to about .04 inches and, preferably, about .025 inches.

As previously discussed, locking elements 116 are spaced along the longitudinal axis "j", Preferably, adjacent locking projections 116 are spaced a longitudinal distance "t" which is sufficient to accommodate at least some of tbe tissue portions displaced by the projecting structure of the locking elements 116. In a preferred embodiment, distance "t" ranges from about .025 inches to about .075 inches and, more preferably, about .050 inches.

The above noted dimensions for cannula sleeve 104 including dimensions "t", "m", "b", and "k" are for a 12 millimeter (mm) cannula sleeve. Other dimensions are also contemplated. Further embodiments comprise 5-10mm cannula sleeves, as well as other sizes. It is also envisioned that other dimensions may be applicable for sleeves of other sizes.

The plurality of locking elements 116 encompass from about 50% to about 95% of the overall longitudinal length of cannula sleeve 104. Preferably, locking elements 116 encompass at least 80% of the longitudinal length of cannula sleeve 104. In this manner, cannula sleeve 104 may be introduced relative to the tissue at various relative locations while ensuring that a sufficient number of locking elements 116 are in contact with the tissue to prevent rehopulsion of the cannula sleeve 104, Locking elements 116 do not extend to flange 106 but are spaced from the flange 106 and cannula housing 102.

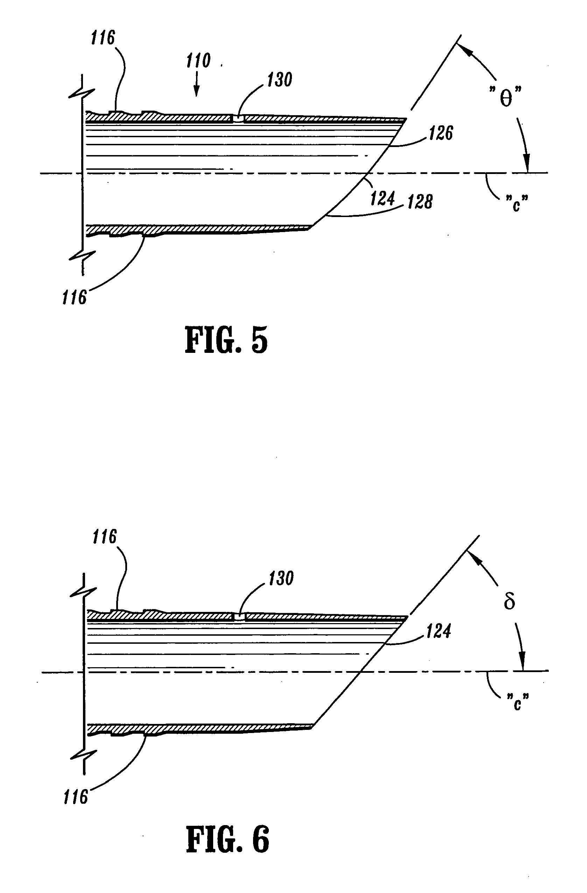

With reference now to Figure 5, the leading or distal end portion 110 of cannula sleeve 104 will be discussed, Leading end portion 110 preferably defines beveled leading end 124. Beveled leading end 124 preferably incorporates first and second end surfaces 126,128 which intersect along a median plane "c" bisecting cannula sleeve 104 and extending along and coincident with longitudinal axis "j". First end surface 126 is preferably arranged at an angle "θ" ranging from about 35° to about 75°, and, more preferably, about 55° relative to the longitudinal axis "j". Second end surface 128 is preferably arranged at an angle "T" ranging from about 25° to about 65°, and more preferably, about 45° relative to the longitudinal axis "j". First and second surfaces 126, 128 may be straight or curved or a combination thereof.

First and second intersecting surfaces 126, 128 provide a generally curved and/or beveled characteristic to leading end 124. This arrangement provides advantages with respect to introduction of cannula sleeve 104, e.g., including a reduction in the insertion force into tissue when the assembled surgical trocar assembly is inserted into tissue. For example, the beveled characteristic reduces the profile of the distal end of cannula sleeve 104 to facilitate initial entry into, and passage through, the tissue, It is also envisioned that leading end 124 may encompass a single linear surface arranged at a desired angle "δ" relative to the longitudinal axis "j" as shown in Figure 6. Alternatively, leading end 124 may be arcuate in nature having a predetermined radius of curvature. As a further alternative, leading end 124 may include a compound curve surface incorporating at least two radii of curvature. Other arrangements are also envisioned.

Outer wall 112 may also incorporate an aperture 130 spaced from leading end 124 and extending completely through the outer wall 112. Aperture 130 permits insufflation gases to exit outer wall 112 and enter the body cavity to maintain or sustain the insufflated state of the body cavity. In addition, aperture 130 prevents the formation of a vacuum upon withdrawal of the obturator or an instrument from the cannula sleeve.

With reference again to Figure 1, trocar assembly 10 may also include a seal assembly 2000 which is preferably releasably mounted to cannula housing 1002. Means for releasably connected seal assembly 2000 to cannula housing 1002 may include a bayonet coupling, friction fit, tongue and groove arrangements, snap-fit, etc. Seal assembly 2000 includes seal housing 2002 and an internal seal which is adapted to form a fluid tight seal about an instrument inserted through the seal assembly 2000. One suitable seal may be the fabric seal disclosed in commonly assigned U.S. Patent No. 6,702,787, filed June 6, 2002, the entire contents of which are incorporated herein by reference. The seal disclosed in certain embodiments of the '787 patent is a flat septum seal having a first layer of resilient material and a second fabric layer juxtaposed relative to the first layer. Further details of the seal may be ascertained by reference to the '787 patent. Seal assembly 2000 may or may not be a component of cannula assembly 1000. Seal assembly 2000 or cannula housing 102 may also incorporate a zero closure valve. The preferred zero closure valve is adapted to open to permit passage of surgical instrumentation and close in the absence of the instrument to prevent escape of the insufflation gases when cannula 100 is not being utilized. For example, the zero closure valve may comprise a flapper valve or duckbill seal.

In use of trocar assembly 10, the abdominal cavity is insufflated. Thereafter, the assembled trocar assembly 10 of Figure 1 is applied to the abdomen whereby obturator tip 1006 of obturator 1000 penetrates the abdomen and peritoneal membrane, and enters the peritoneal cavity. Obturator 1000 is removed from cannula 100. Cannula 100 provides access to the tissue and/or organs within the peritioned cavity for the introduction of instrumentation and/or a laparoscope. By virtue of locking elements 106, cannula sleeve 104 is retained within the tissue in the aforedescribed manner.

While the invention has been particularly shown, and described with reference to the preferred embodiments, it will be understood by those skilled in the art that various modifications and changes in form and detail may be made therein without departing from the scope and spirit of the invention. Accordingly, modifications such as those suggested above, but not limited thereto, are to be considered within the scope of the invention.

## Claims

1. A surgical access apparatus, which comprises:
an access member dimensioned for positioning within body tissue, the access member including an outer wall defining a longitudinal opening along a longitudinal axis of the access member, the outer wall including at least one locking element projecting radially outwardly from the outer wall, the at least one locking element defining first, second and third surfaces, the first surface being a leading surface and arranged at a leading angle relative to the longitudinal axis to permit insertion of the access member into the body tissue, the second surface extending from the first surface, the thirds surface extending from the second surface and being a trailing surface arranged at a trailing angle relative to the longitudinal axis to resist removal of the access member from the body tissue.

2. The surgical access apparatus according to claim 1, wherein the outer wall of the access member is transparent.

3. The surgical access apparatus according to claim 1 or 2, wherein said at least one locking element is present as a plurality of locking elements.

4. The surgical access apparatus according to claim 3, wherein adjacent ones of said locking elements are longitudinally spaced relative to the longitudinal axis.

5. The surgical access apparatus according to any one of the preceding claims, wherein the leading angle of the first surface is in a range of from 5° to 30° relative to the longitudinal axis.

6. The surgical access apparatus according to claim 6, wherein the leading angle is in a range of from 14° to 16° relative to the longitudinal axis.

7. The surgical access apparatus according to any one of the preceding claims, wherein the trailing angle is substantially transverse to the longitudinal axis.

8. The surgical access apparatus according to any one of the preceding claims, wherein the access member includes a beveled leading end.

9. The surgical access apparatus according to claim 8, wherein the beveled leading end includes first and second intersecting surfaces.

10. The surgical access instrument according to claim 9, wherein the first and second intersecting surfaces of the beveled leading end intersect along a median bisecting plane of the access member.

11. The surgical access instrument according to any one of claims 8 to 10, wherein the beveled leading end has an arcuate characteristic.

12. A surgical access instrument, comprising an access member dimensioned for positioning within body tissue and defining a longitudinal axis, the access member having leading and trailing ends, the leading end including first and second intersecting surface arranged with respect to the longitudinal axis at first and second oblique angles.

13. The surgical access instrument according to claim 12, wherein the access member includes an outer wall defining a longitudinal opening extending along the longitudinal axis.

14. The surgical access instrument according to claim 12 or 13, wherein the first and second interesting surfaces of the lading end intersect along a median bisecting plane of the access member.

15. The surgical access instrument according to claim 12, 13 or 14, wherein the first surface of the leading end defines an angle in a range of from 35° to 75° relative to the longitudinal axis and wherein the second surface of the leading end defines an angle in a range of from 25° to 65° relative to the longitudinal axis.

16. The surgical access instrument according to claim 15, wherein the first surface of the leading end defines an angle in a range of from 50° to 60° relative to the longitudinal axis.

17. The surgical access instrument according to claim 15 or 16, wherein the second surface of the leading end defines an angle in a range of from 50° to 50° relative to the longitudinal axis.

18. The surgical access instrument according to claim 13 or any one of claims 14 to 17 as dependent on claim 13, wherein the outer wall of the access member includes at least one locking projection projecting radially outwardly from the outer wall, the at least one locking projection defining first, second and third surfaces, the first surface being a leading surface and arranged at a leading angle relative to the longitudinal axis to permit insertion of the access member in the body tissue, the second surface extending from the first surface, the third surface extending from the second surface and being a trailing surface arranged at a trailing angle relative to the longitudinal axis to resist removal of the access member from the body tissue.

19. The surgical access instrument according to claim 18, wherein said at least one locking projection is present as a plurality of locking projections.

20. The surgical access instrument according to claim 19, wherein adjacent locking projections are longitudinally spaced relative to the longitudinal axis.
